Europäisches Patentamt

European Patent Office

Office européen des brevets

(11) Numéro de publication: **0 204 680**
**B1**

(12) # FASCICULE DE BREVET EUROPEEN

(45) Date de publication du fascicule du brevet:
**03.05.89**

(51) Int. Cl.⁴: **A61K 39/29**

(21) Numéro de dépôt: **86870069.1**

(22) Date de dépôt: **21.05.86**

(54) Procédé d'isolement et de purification de l'antigène de surface de l'hépatite B.

(30) Priorité: **30.05.85 US 739415**

(43) Date de publication de la demande:
**10.12.86 Bulletin 86/50**

(45) Mention de la délivrance du brevet:
**03.05.89 Bulletin 89/18**

(84) Etats contractants désignés:
**AT BE CH DE FR GB IT LI LU NL SE**

(56) Documents cités:
**EP-A- 0 155 007**

**ACTA PATH. MIRCOBIOL. SCAND., section B, vol. 80, 1972, pages 935,936; J.C. SIEBKE et al.: "On the adsorption of Australla antigen to a colloidal silica, and some possible applications"**
**CHEMICAL ABSTRACTS, vol. 86, no. 7 14 février 1977, page 374, résumé no. 41604u, Columbus, Ohio, US; J. PILLOT et al.: "Optimal conditions for elution of hepatitis B antigen after absorption onto colloidal silica"**
**NUCLEIC ACIDS RESEARCH, vol. 11, no. 9, 11 mai 1983, pages 2745-2763, IRL Press Ltd., Oxford, GB; R.A. HITZEMAN et al.: "Expression of hepatitis B virus surface antigen in yeast"**

(73) Titulaire: **Smith Kline - RIT Société anonyme dite:, rue du Tilleul, 13, B-1320 Genval (Rixensart)(BE)**

(72) Inventeur: **van Wijnendaele, Frans, Teckerstraat, 12, B-3052 Ottenburg(BE)**
Inventeur: **Simonet, Guy, Chaussée de Wavre, 14, B-5920 Perwez(BE)**

(74) Mandataire: **Tasset, Gérard, SMITHKLINE - RIT rue de l'Institut, 89, B-1330 Rixensart(BE)**

**Description**

Domaine de l'invention

La présente invention concerne un procédé perfectionné d'isolement et de purification de l'antigène de surface de l'hépatite B (HBsAg) dans un but vaccinal et la préparation des vaccins qui en dérivent.

L'antigène HBsAg obtenu par le procédé de l'invention se présente sous la forme de particules d'HBsAg mesurant de 17 à 20 nm. Il présente un intérêt pour la préparation de vaccins.

Arrière plan technologique

L'hépatite B est une maladie virale mondialement répandue et potentiellement mortelle. Bien que son agent étiologique – c'est-à-dire le virus de l'hépatite B – ait été isolé depuis longtemps au départ du sang de malades atteints de la maladie, le développement d'un vaccin commercial contre l'hépatite B a rencontré d'importantes difficultés: le virus de l'hépatite B se présente sous la forme d'une particule de 42 nm appelée particule de Dane comprenant (a) un noyau contenant le génome viral lié à la protéine nucléocapsidique et contenant l'antigène nucléocapsidique (HBcAg) qui n'est pas immunogène et (b) une enveloppe contenant l'antigène de surface (HBsAg) immunogène. L'HBsAg est formé d'un groupe de structures macromoléculaires complexes morphologiquement hétérogènes; il contient des protéines, des hydrates de carbone, des glycoprotéines et des lipides, les constituants principaux étant la phosphatidylcholine, l'ester de cholestéryle, le cholestérol et les triglycérides. L'infection par le virus de l'hépatite B (HBV) aboutit non seulement à la production de particules de Dane mais également à une surproduction de particules de 22 nm et de filaments contenant les éléments de l'enveloppe de surface. On sait que ces particules de 22 nm sont environ 1000 fois plus immunogènes que la protéine HBsAg monomère mais la reproduction et l'expression du virus de l'hépatite B (HBV) a été entravée pendant longtemps par l'absence de systèmes de culture cellulaire appropriée pour la multiplication du virus in vitro. L'absence de systèmes de culture in vitro dans lesquels le virus peut se propager efficacement a conduit au développement de recherches orientées soit vers la synthèse de molécules du type HBsAg soit vers l'expression de l'HBsAg dans d'autres systèmes hôtes, en utilisant la technique dite d'ADN recombinant. Par exemple, l'expression de l'HBsAg a été signalée au départ de cellules de levure transformées à l'aide de vecteurs d'ADN portant le gène de l'HBsAg et on sait que l'HBsAg synthétisé dans la levure est assemblé en particules présentant un diamètre de 17 à 20 nm et possédant des propriétés analogues aux particules de 22 nm secrétées par les cellules humaines (P. VALENZUELA et al. Nature, 298, 347–350; 1982 et R.A. HITZEMANN et al. Nucl. Ac. Res. 11; 2745–2763; 1983). La production d'HBsAg par la technique dite d'ADN recombinant dans des cultures de cellules produites génétiquement, par exemple des cultures de souches de levure produites génétiquement, est bien connue dans la technique (voir par exemple la demande de brevet européen publiée sous le numéro 0 106 828; W.J. McALEER et al. dans Nature 307; 178–180, 1984 et C.E. CARTY et al. dans l'abrégé 030 de l'Assemblée annuelle de l'American Society for Microbiology 1984, 84ème assemblée, 4–9 mars, 1984).

De nombreux articles et brevets ont également paru, décrivant des procédés d'extraction et de purification de l'HBsAg au départ de plasma, de sérum ou d'autres composants sanguins ou au départ d'autres liquides biologiques ou de cultures de cellules produites génétiquement.

Le procédé de la présente invention est applicable aux cellules de levure qui sont produites génétiquement et qui sont productrices d'HBsAg.

L'extraction et la purification de l'HBsAg au départ d'une culture de cellules de levure produites génétiquement qui s'est développée jusqu'à une densité cellulaire satisfaisante nécessitent généralement 3 étapes successives, à savoir:

(1) l'extraction de l'HBsAg de l'intérieur de la cellule;
(2) l'enrichissement du milieu en HBsAg;
(3) l'élimination substantielle de tous les contaminants du milieu.

Dans la plupart des procédés, l'étape (1) est réalisée par l'action de forces mécaniques telles que les forces de cisaillement (par exemple la cellule de pression X – "X press" – ou la cellule de pression de French) ou par agitation avec des billes de verre éventuellement en présence d'un détergent. L'utilisation d'un détergent non ionique (Triton X-100) a été signalée par K. MURRAY et al. dans The EMBO J. 3; 645–650; 1984 et par R.A. HITZEMANN et al.; loc. cit.

Il a également été signalé que l'incubation de l'HBsAg en présence de détergents brise la particule. Dans cet ordre d'idée:
– le brevet GB 2 093 039 décrit le traitement de l'HBsAg par un détergent non ionique (Triton X-100) pour former un mélange polypeptidique que l'on introduit sur une solution tampon aqueuse contenant un gradient de saccharose et l'isolement d'une fraction substantiellement exempte de détergent et contenant des micelles du mélange polypeptidique.
– le brevet EP 0 005 864 décrit la mise en contact d'une masse antigénique contenant l'antigène de surface de l'hépatite B (HBsAg) et l'antigène nucléocapsidique (HBcAg) d'une dimension particulaire d'au moins 20 nm avec un détergent (p.ex. le Tween 20 ou 80) suivie du traitement du détergent contenant la composition antigénique par une solution aldéhydique aqueuse. Le produit obtenu est une masse sous-particulaire présentant une dimension particulaire inférieure à 20 nm et plus particulièrement inférieure à 5 nm.

– la demande de brevet japonais 53 104 724 (résumé Derwent 75324 A) décrit un procédé dans lequel les particules d'HBsAg sont chauffées en présence (1) d'un détergent non ionique (c'est-à-dire l'alcoylphé-nyl polyoxyéthylène doté de 7 à 10 chaînes oxyéthylène, (2) d'un agent dénaturant de protéines (c'est-à-dire l'urée ou la guanidine) et (3) éventuellement d'un agent réducteur. Le produit obtenu présente une dimension particulaire comprise entre 10 et 20 nm.

– la demande de brevet japonais 50 160 420 (résumé Derwent 27420 A) décrit un procédé dans lequel l'HBsAg est traité par différents surfactants pour former des produits sous-unitaires.

– la demande de brevet de la République Fédérale d'Allemagne 2 611 723 décrit un procédé dans lequel des particules sphériques d'HBsAg sont obtenues en chauffant l'antigène en présence d'un surfactant dégraissant.

– le brevet des Etats-Unis d'Amérique 4 113 712 décrit des particules d'HBsAg constituées de sousunités polypeptidiques simples préparées par chauffage de l'HBsAg dans une solution isotonique de chlorure de sodium à un pH voisin de la neutralité et contenant un détergent pouvant provoquer une délipidation.

– le brevet des Etats-Unis d'Amérique 4 481 189 décrit un procédé dans lequel du plasma sanguin ou un dérivé de plasma est stérilisé par contact avec un détergent (p.ex. un détergent non ionique).

Les étapes (2) et (3) comprennent bon nombre de techniques, parmi lesquelles l'adsorption/désorption à l'aide de silice colloïdale, plus particulièrement l'"AEROSIL" (un produit vendu par DEGUSSA, Francfort, RFA).

Une technique de ce genre est décrite dans les références suivantes:

– W.J.M. DUIMEL et al. dans Vox Sang. 23; 249–255, 1972;
– J. PILLOT et al. dans J. Clin. Microbiol. 4; 205–207; 1976 et dans Molec. Immunol. 21; 53–60; 1984;
– F. BARIN et al. dans Ann. Microbiol. (Inst. Pasteur) 129 B; 87–100; 1978

qui montrent que, même dans les meilleures conditions opératoires, on ne récupère qu'une fraction (c'est-à-dire environ 60%) de l'HBsAg adsorbé, (les meilleurs résultats étant obtenus par élution au moyen d'un tampon de force ionique faible) à moins d'ajouter une certaine quantité de désoxycholate à l'éluant.

Dans une autre étude (SIEBKE et al. dans Acta Path. Microbiol. Scand. Section B; 80; 935–936; 1972), il a été démontré que l'antigène australien est partiellement élué de l'Aérosil si on utilise 2% de Tween 80 et qu'il est également faiblement adsorbé en présence du détergent.

<u>Description de l'invention</u>

Lorsqu'on applique la technique connue (à savoir celle qui implique l'adsorption sur gel de silice et la désorption à l'aide d'un tampon désoxycholate), à un surnageant provenant du lysat de cellules productrices d'HBsAg après culture et obtenu par addition d'un détergent non ionique, on a remarqué que, non seulement il n'est pas possible d'obtenir un surnageant clair de cellules éclatées mais que l'utilisation du tampon désoxycholate en vue de la désorption à partir de silice colloïdale n'aboutit qu'à une désorption partielle de l'HBsAg; de plus, il se produit une dégradation de la particule de levure, comme le démontre la chromatographie sur Sépharose 4B-Cl, puisqu'aucun antigène associé à l'hépatite n'est ensuite détecté à l'emplacement habituel (Kd: 0,25).

Par centrifugation sur gradient de saccharose, on a également découvert que, lorsque l'éclatement des cellules de levure est réalisé en présence de 0,5% (p/v) de Triton X-100, la structure de la particule d'HBsAg ne reste pas intacte, tandis que lorsqu'on remplace le Triton X-100 par 0,5% (v/v) d'un polysorbate (p.e. le polysorbate 20), on améliore de façon substantielle le rendement en HBsAg intact.

En outre, on a également trouvé maintenant que, lorsque le surnageant de cellules de levure éclatées en présence d'un détergent non ionique du type polysorbate (p.ex. le polysorbate 20 ou 80) est additionné d'urée – jusqu'à une concentration finale de 2 à 6 M – on obtient (p.ex. par agitation à température ordinaire pendant 30 minutes) une solution partiellement clarifiée.

Par ailleurs, on a également trouvé que, lors du traitement de la solution clarifiée avec de l'Aérosil, on peut désorber complètement l'HBsAg de la solution en employant un système tampon de force ionique faible, à condition de remplacer l'additif du type désoxycholate utilisé jusqu'ici par un additif du type urée/détergent non ionique.

L'intégralité de la désorption de l'HBsAg en procédant de cette manière a été démontrée par l'absence d'une bande 22 K dans la silice colloïdale désorbée et on a confirmé que l'HBsAg isolé est assemblé en particules d'environ 17 à 20 nm, lesquelles induisent un taux élevé d'anticorps HBsAg par administration parentérale à des animaux de laboratoire.

En conséquence, un premier objectif de la présente invention est de fournir un procédé perfectionné de préparation de l'antigène de surface de l'hépatite B applicable à des fins vaccinales au départ d'une culture de cellules de levure produites génétiquement et ayant exprimé l'HBsAg, lequel procédé consiste à faire éclater les cellules en présence d'un détergent non ionique – plus particulièrement un polysorbate (p.ex. 0,3% v/v de polysorbate 20 ou 80) et à y ajouter de l'urée jusqu'à concentration finale de 2 à 6 M, de façon à obtenir une solution clarifiée en HBsAg (p.ex. par agitation à température ordinaire pendant 30 minutes), à adsorber l'HBsAg sur de la silice colloïdale (comme l'AEROSIL) (p.ex. par agitation à 4°C pendant une nuit), à désorber l'HBsAg à 37°C à un pH compris entre 8 et 9,5 à l'aide d'un tampon de force ionique faible (p.ex. 10 mM) additionné d'urée à une concentration finale de 8 M et d'un détergent ionique, plus particulièrement un polysorbate (p.ex. le polysorbate 20 ou 80 à une concentration finale de 0,1 à 1% v/v).

Le désorbat contient des particules d'HBsAg purifiées qui peuvent être traitées ultérieurement suivant n'importe quelle technique bien connue dans la pratique, p.ex. l'ultrafiltration, la chromatographie sur agarose, la précipitation à l'aide de sulfate de dextran/chlorure de calcium et la dialyse.

L'invention est illustrée par les exemples suivants.

## EXEMPLE 1

Un culot de cellules (d'un poids sec de 80 g), provenant de la culture d'une souche de levure produite génétiquement productrice d'HBsAg et qui s'est développée jusqu'à une production de 30 g de cellules (en poids sec) par litre de culture est mise en suspension dans 150 ml d'une solution aqueuse de $Na_2HPO_4$ (7,098 g/l). Cette suspension est additionnée de 3 ml d'une solution d'EDTA à 4% (p/v), 0,9 ml de polysorbate 20 et 90 ml d'isopropanol contenant 60 mg de fluorure de phénylméthylsulfonyle (FPMS). Le pH est ajusté à 8,1 avec NaOH (10% p/v dans l'eau). La suspension est refroidie dans un bain de glace et brisée par deux passages à travers un homogénéiseur à billes de verre réfrigéré. L'homogénat est ensuite centrifugé pendant 30 minutes à 13000 g.

Le surnageant contenant l'extrait brut (160 ml) est additionné d'urée jusqu'à obtention d'une concentration finale 4 M en urée et agité pendant 30 minutes à température ordinaire de manière à séparer les particules d'HBsAg des membranes cellulaires de levure contaminantes et on y ajoute de l'AEROSIL® 380 (2,5% p/v) prélavé dans un tampon phosphate 50 mM ($NaH_2PO_4/NaOH$), pH 7,2, contenant 0,3% (v/v) de polysorbate 20 et de l'urée 4 M. La solution est agitée à 4°C pendant une nuit de manière à permettre une adsorption maximale des particules d'HBsAg sur les particules de silice.

La silice colloïdale est ensuite centrifugée pendant 15 minutes à 6500 g et lavée à deux reprises avec des fractions de 250 ml de NaCl à 10% (p/v). La silice lavée est mise en suspension dans 80 ml de tampon borate 10 mM, pH 9, ($Na_2B_4O_7/HCl$), additionnée de 0,5% (v/v) de polysorbate 20 d'urée (à une concentration finale 8 M) et la suspension est agitée à 37° pendant 4 heures.

La silice colloïdale désorbée est ensuite centrifugée pendant 30 minutes à 27000 g de façon à séparer une quantité maximale de particules de silice colloïdale.

La centrifugation par gradient dans un gradient de saccharose ainsi que la microscopie électronique montrent que l'antigène est présent sous la forme de particules ayant un diamètre d'environ 17–20 nm.

Le désorbat est concentré jusqu'à 15 ml dans un appareil AMICON DC (un appareil vendu par AMICON CORP. DANVERS, MA, EUA) muni d'une cartouche ayant un cut-off de 100000 daltons et appliqué sur une colonne (diamètre 2,5 cm x 100 cm) de SEPHAROSE 4B-Cl (un gel d'agarose fabriqué et vendu par PHARMACIA FINE CHEMICALS, Uppsala, Suède) équilibrée dans un tampon trométamol/HCl 10 mM (pH 8) contenant 0,3% (v/v) de polysorbate 20.

Le pic contenant l'antigène (Kd = 0,25) est traité par du sulfate de dextran (PM 50 000) à une concentration finale de 0,1% (p/v) et par du $CaCl_2$ à une concentration finale de 40 mM.

L'excès de sulfate de dextran est précipité par dialyse contre un solution de $BaCl_2$ 40 mM dans du tampon trométamol/HCl 10 mM (pH 8). Le dialysat (30 ml) est soumis à une centrifugation par gradient sur CsCl et le pic contenant l'HBsAg (9 ml) est recueilli.

La purification de l'HBsAg au cours des différentes étapes du procédé susmentionné est résumée sommairement dans le tableau I.

L'analyse électrophorétique (SDS-PAGE) montre que le produit final est pur à raison d'au moins 98% en HBsAg.

Tableau I

| Fraction | HBsAg[x] (µg) | Protéines (mg) | Polysac- charides (mg) | Acides nucléiques (µg) |
|---|---|---|---|---|
| Surnageant en extrait brut | 1790 | 954 | 353 | 5300 |
| Désorbat concentré | 1000 | 49 | 23,5 | 700 |
| Pic Kd 0.25 | 328 | 20 | 15 | 780 |
| Surnageant sulfate de dextran | 432 | 2,8 | 32 | 724 |
| BaCl$_2$ | 432 | 2,2 | 17,5 | 790 |
| Gradient CsCl | 270 | 0,306 | 0,378 | 108 |

(x) Par RIA (Radio immuno dosage)

## EXEMPLE 2

Un culot de cellules (d'un poids sec de 80 g), provenant de la culture d'une souche de levure produite génétiquement productrice d'HBsAg et qui s'est développée jusqu'à une production de 30 g de cellules (en poids sec) par litre de culture est mise en suspension dans 150 ml d'une solution aqueuse de Na$_2$HPO$_4$ (7,098 g/l). Cette suspension est additionnée de 3 ml d'une solution d'EDTA à 4% (p/v), 0,9 ml de polysorbate 20 et 9 ml d'isopropanol contenant 60 mg de fluorure de phénylméthylsulfonyle (FPMS). Le pH est ajusté à 8,1 avec NaOH (10% p/v dans l'eau). La suspension est refroidie dans un bain de glace et brisée par 2 passages à travers un homogénéiseur à billes de verre réfrigéré. L'homogénat est ensuite centrifugé pendant 30 minutes à 13000 g.

Le surnageant contenant l'extrait brut (160 ml) est additionné d'urée jusqu'à obtention d'une concentration finale 4 M en urée et agité pendant 30 minutes à température ordinaire de manière à séparer les particules d'HBsAg des membranes cellulaires de levure contaminantes et on y ajoute de l'AEROSIL® 380 (2,5% p/v) prélavé dans un tampon phosphate 50 mM (NaHPO$_2$/Na$_4$OH) pH 7,2, contenant 0,3% (v/v) de polysorbate 20 et de l'urée 4 M. La solution est agitée à 4°C pendant une nuit de manière à permettre une adsorption maximale des particules d'HBsAg sur les particules de silice.

La silice colloïdale est ensuite centrifugée pendant 15 minutes à 6500 g et lavée à deux reprises avec des fractions de 250 ml de NaCl à 10% (p/v).

La silice lavée est mise en suspension dans 80 ml de tampon borate 10 mM, pH 9 (Na$_2$B$_4$O$_7$/HCl) additionnée de 0,5% (v/v) de polysorbate 20 et par de l'urée (à une concentration finale 8 M) et la suspension est agitée à 37° pendant 4 heures.

La silice colloïdale désorbée est ensuite centrifugée pendant 30 minutes à 27000 g de façon à séparer une quantité maximale de particules de silice colloïdale.

Le désorbat est dialysé contre un tampon trométamol 10 mM (pH 7,5) dans un appareil AMICON DC muni d'une cartouche ayant un cut-off de 100000 daltons afin de dialyser l'urée et appliqué, à raison d'un débit de 10 ml par heure, sur une colonne de 25 ml d'hexylagarose (un produit fabriqué et vendu par PHARMACIA FINE CHEMICALS, Uppsala, Suède) équilibrée dans un tampon trométamol 10 mM/HCl (pH 7,5).

La colonne est ensuite lavée avec du tampon trométamol 10 mM (pH 7,5) et éluée avec un mélange 50/50 (v/v) d'éthylène glycol et de tampon trométamol/HCl 10 mM (pH 7,5) additionné de NaCl à une concentration finale 1 M et ensuite avec un tampon trométamol/HCl 10 mM (pH 7,5) additionné d'urée à une concentration finale 4 M.

La purification de l'HBsAg au cours des différentes étapes du procédé ci-dessus est résumée sommairement dans le tableau II.

Tableau II

| Fraction | HBsAG[1] (µg) | Protéines (mg) | Polysac-charides (mg) | Acides nucléiques (µg) |
|---|---|---|---|---|
| Extrait brut | 3,5 | 2224 | 517 | 17,9 |
| Désorbat | 2,1 | 86,4 | 30,1 | 2,1 |
| Eluat éthylène glycol/NaCl | 1,4 | 1,10 | 0,05 | ND[2] |
| Eluat urée | 0,7 | 0,72 | 0 | ND[2] |

(1) Par RIA (Radio immuno dosage)
(2) ND = non détectable

L'analyse électrophorétique (SDS-PAGE) montre que le produit final est pur à raison d'au moins 97% en HBsAg.

EXEMPLE 3

La technique est celle de l'exemple 2, mais on remplace l'hexylagarose qui y est spécifiée par 25 ml de phénylagarose (un produit fabriqué par PHARMACIA FINE CHEMICALS, Uppsala, Suède).

Le processus de purification de l'HBsAg et le taux en HBsAg dans le produit final sont analogues à ceux indiqués à l'exemple 2.

EXEMPLE 4

La solution d'HBsAg obtenue à la fin de l'exemple 1 a été ajustée à un contenu protéinique de 10 µg par ml par addition de NaCl, de tampon phosphate ($Na_2HPO_4/NaH_2PO_4$) et d'ALHYDROGEL® (un gel d'hydroxyde d'aluminium fabriqué et vendu par SUPERPHOS Export Co., Copenhague, Danemark) jusqu'à concentrations finales respectives de 0,9% (p/v), 20 mM et 0,15% (p/v) en $Al(OH)_3$, le pH final étant de 6,9.

La préparation a été stérilisée et répartie dans des fioles en verre de 2 ml contenant chacune une dose unitaire d'un ml de vaccin.

EXEMPLE 5

Différentes dilutions des doses unitaires de la préparation vaccinale de l'exemple 4 ont été administrées par voie intrapéritonéale à 5 groupes de souris Swiss âgées de cinq semaines.

Vingt-huit jours après l'administration, les animaux ont été saignés et les titres sériques anti-HBs ont été déterminés pour chaque échantillon suivant la méthode dite AUSAB® (AUSAB est la marque déposée pour une trousse d'analyse fabriquée et vendue par ABBOTT Diagnostic Products GmbH, Wiesbaden, RFA).

En même temps et en suivant le même schéma de vaccination, les mêmes dilutions des doses unitaires de la préparation vaccinale, obtenus suivant la technique de l'exemple 4 mais en utilisant un antigène préparé selon la technique de l'exemple 1 sans l'étape à la silice colloïdale, ont également été administrées aux 5 groupes de souris Swiss âgées de cinq semaines.

Les résultats de cet essai comparatif sont résumés sommairement au tableau III dans lequel (A) désigne le vaccin obtenu suivant la technique de l'invention alors que (B) désigne le vaccin obtenu suivant le procédé sans l'étape à la silice colloïdale.

Tableau III

| Préparation vaccinale | Protéines (mg) | Nombre de souris | Taux de séro-conversion (%) | DE$_{50}$ (ng RIA) |
|---|---|---|---|---|
| A | ,039 | 10 | 0 | |
| | ,156 | 10 | 60 | |
| | ,925 | 10 | 90 | 180 |
| | 2,5 | 10 | 90 | |
| | 10 | 6 | 83 | |
| B | ,039 | 10 | 10 | |
| | ,156 | 10 | 10 | |
| | ,625 | 10 | 20 | 1954 |
| | 2,4 | 10 | 50 | |
| | 10 | 10 | 80 | |

DE$_{50}$: dose efficace chez 50% des animaux traités
RIA: Radio Immuno dosage

## Revendications

1. Procédé d'isolement et de purification de l'antigène de surface de l'hépatite B par traitement, avec de la silice colloïdale, du surnageant provenant de cellules de levure modifiées génétiquement ayant produit ledit antigène et éclatées en présence d'un détergent non ionique, caractérisé en ce qu'on fait éclater les cellules en présence d'un polysorbate, on clarifie le surnageant par addition d'urée, on adsorbe l'antigène sur la silice colloïdale et on désorbe l'antigène de celle-ci avec un tampon de force ionique faible (pH 8–9,5) additionné d'urée et d'un détergent non ionique.

2. Procédé suivant la revendication 1 dans lequel la clarification du surnageant est réalisée par addition d'urée jusqu'à concentration finale comprise entre 2 et 6 M.

3. Procédé suivant l'une quelconque des revendications 1 et 2, dans lequel l'étape de clarification est réalisée à 4°C.

4. Procédé suivant l'une quelconque des revendications 1 à 3, dans lequel le tampon de force ionique faible est 10 mM.

5. Procédé suivant l'une quelconque des revendications 1 à 4, dans lequel l'urée est ajoutée au tampon de force ionique faible jusqu'à concentration finale 8 M.

6. Procédé suivant l'une quelconque des revendications 1 à 5, dans lequel la désorption est réalisée à 37°C.

7. Procédé suivant l'une quelconque des revendications 1 à 6, dans lequel le détergent non ionique ajouté au tampon de force ionique faible est un polysorbate.

8. Procédé suivant la revendication 7, dans lequel le polysorbate est un polysorbate 20 ou 80.

9. Procédé suivant l'une quelconque des revendications 7 et 8, dans lequel la concentration finale en polysorbate est comprise entre 0,1 et 1% (v/v).

## Patentansprüche

1. Verfahren zur Isolierung und Reinigung des Hepatitis B Oberflächenantigens, bei dem man den Überstand von genetisch modifizierten Hefezellen, die das Antigen produzieren und in Gegenwart eines nicht-ionischen Tensids aufgeschlossen werden, mit kolloidalem Kieselgel behandelt, dadurch gekennzeichnet, daß man die Zellen in Gegenwart eines Polysorbats aufschließt, den Überstand durch Zugabe von Harnstoff klärt, das Antigen an kolloidalem Kieselgel adsorbiert und das Antigen von dort mit einem schwach ionischen Puffer (pH 8–9,5), dem Harnstoff und ein nicht-ionisches Tensid zugesetzt ist, desorbiert.

2. Verfahren nach Anspruch 1, bei dem die Klärung des Überstands durch Zugabe von Harnstoff bis zu einer Endkonzentration zwischen 2 und 6 M erfolgt.

3. Verfahren nach einem der Ansprüche 1 und 2, bei dem der Klärungsschritt bei 4°C durchgeführt wird.

4. Verfahren nach einem der Ansprüche 1 bis 3, bei dem der schwach ionische Puffer 10 mM ist.

5. Verfahren nach einem der Ansprüche 1 bis 4, bei dem der Harnstoff dem schwach ionischen Puffer bis zu einer Endkonzentration von 8 M zugesetzt wird.

6. Verfahren nach einem der Ansprüche 1 bis 5, bei dem die Desorption bei 37°C erfolgt.

7. Verfahren nach einem der Ansprüche 1 bis 6, bei dem das nicht ionische Tensid, das dem schwachionischen Puffer zugesetzt wird, ein Polysorbat ist.

8. Verfahren nach Anspruch 7, bei dem das Polysorbat ein Polysorbat 20 oder 80 ist.

9. Verfahren nach einem der Ansprüche 7 und 8, bei dem die Polysorbat-Endkonzentration im Bereich von 0,1 und 1% (V/V) liegt.

**Claims**

1. A process for the isolation and purification of hepatitis B surface antigen by treatment with colloïdal silica of the supernatant of engineered yeast cells having produced said antigen and disrupted in the presence of a non ionic detergent which comprises disrupting the cells in the presence of a polysorbate, clarifying the supernatant by addition of urea, adsorbing the antigen on the colloïdal silica, desorbing therefrom the antigen with a low ionic buffer (pH 8–9.5) supplemented with urea and with a non ionic detergent.

2. A process according to claim 1 wherein the clarification of the supernatant is performed by addition of urea up to a final concentration comprised between 2 and 6 M.

3. A process according to claim 1 or 2 wherein the clarification step is performed at 4°C.

4. A process according to anyone of claims 1 to 3 wherein the low ionic strength buffer is 10 mM.

5. A process according to anyone of claims 1 to 4 wherein urea is supplemented to the low ionic buffer up to a final concentration 8 M.

6. A process according to anyone of claims 1 to 5 wherein the desorption is performed at 37°C.

7. A process according to anyone of claims 1 to 6 wherein the non ionic detergent supplemented to the low ionic strength buffer is a polysorbate.

8. A process according to claim 7 wherein the polysorbate is polysorbate 20 or 80.

9. A process according to claim 7 wherein the final concentration in polysorbate is comprised between 0.1 and 1% (v/v).